# FASCICULE DE BREVET EUROPEEN

(11) **EP 2 200 983 B1**
(45) Date de publication et mention de la délivrance du brevet: **28.12.2011**
(21) Numéro de dépôt: 08870986.0
(22) Date de dépôt: 12.09.2008
(51) Int. Cl.: C07D 211/78, C07C 249/04, C07C 249/08, C07C 251/38, C07C 251/44

(54) **PROCEDE DE PREPARATION D'UNE PIPERIDINE DISUBSTITUEE ET INTERMEDIAIRES**
VERFAHREN ZUR HERSTELLUNG VON DISUBSTITUIERTEM PIPERIDIN UND ZWISCHENPRODUKTEN DAVON
METHOD FOR PREPARING DISUBSTITUTED PIPERIDINE AND INTERMEDIATES

(30) Priorité: 14.09.2007 FR 0706449
(43) Date de publication de la demande: 30.06.2010
(73) Titulaire: NOVEXEL, 93230 Romainville (FR)
(72) Inventeur: PRIOUR, Alain, F-75004 Paris (FR); BONNET, Alain, F-02400 Chateau-Thierry (FR); ODDON, Gilles, F-69740 Genas (FR); MAZURIE, Alain, F-93410 Vaujours (FR)
(74) Mandataire: Hirsch & Associés
(86) Numéro de dépôt international: PCT/FR2008/001280
(87) Numéro de publication internationale: WO 2009/090320

(56) Documents cités:
- WO-A-02/10172
- WO-A-02/100860
- WO-A-03/063864
- WO-A-2004/052891

## Description

L'invention a pour objet un procédé de préparation d'une pipéridine 2,5 disubstituée et de nouveaux intermédiaires.

La demande WO 02/10172 décrit des nouveaux composés azabicycliques utiles comme médicaments dans le domaine anti-bactérien et leur préparation mettant en oeuvre des composés intermédiaires de formule (A) : 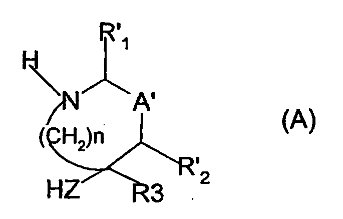 dans laquelle R'₁, R'₂, R₃, Z et n sont définis comme indiqué dans ladite demande, et notamment, parmi ces intermédiaires, une pipéridine de formule (A₁): 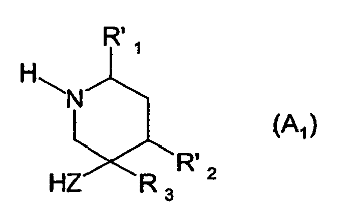 correspondant à un composé de formule (A) dans laquelle n = 1 et A' = CH₂.

Parmi les composés de formule (A₁), le composé répondant à la formule (I) ci-dessous présente un intérêt particulier : 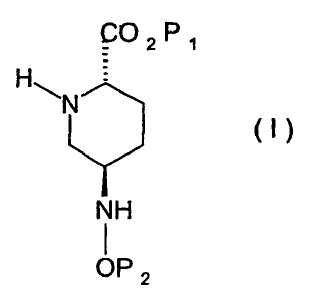 P₁ et P₂ représentent des groupements protecteurs des fonctions acide carboxylique et oxyamine connus de l'homme du métier et notamment ceux cités dans la demande WO 02/10172.

Le composé de formule (I) se présente sous la forme d'un mélange d'isomères (2S, 5R) et (2S,5S).

Le composé de formule (I) peut être obtenu comme décrit dans la demande WO 02/10172, notamment à l'exemple 32, au départ de l'acide cis-5-hydroxy-pipéridine-2-carboxylique protégé.

La présente invention a pour objet un nouveau procédé de préparation du composé de formule (I), caractérisé en ce que l'on traite le composé de formule (b): 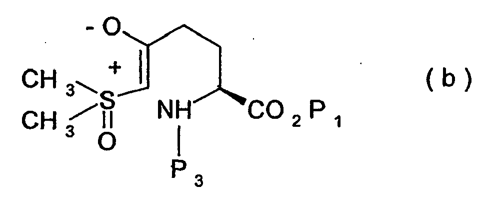 dans laquelle P₁ et P₃ représentent des groupements protecteurs de la fonction acide carboxylique et de l'azote, avec un réactif générant HCl, pour obtenir le composé de formule : 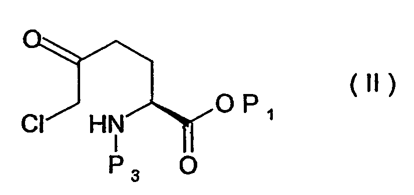 dans laquelle P₁ et P₃ sont définis comme précédemment, que l'on traite sans l'isoler par un dérivé d'hydroxylamine, pour obtenir le composé de formule : 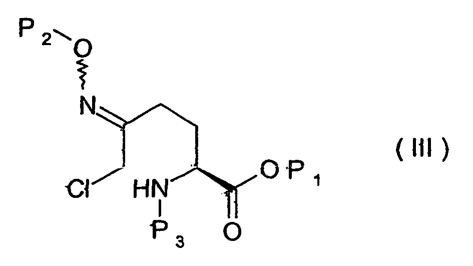 dans laquelle P₁ et P₃ sont définis comme précédemment et P₂ représente un groupement protecteur de l'oxime, dont on déprotège l'amine par action d'un acide, pour obtenir le composé de formule : 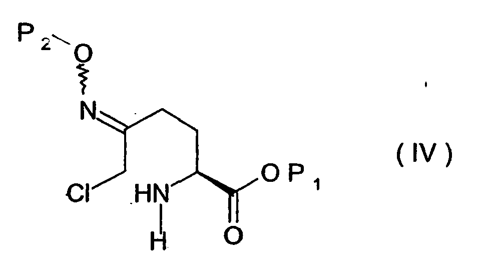 dans laquelle P₁ et P₂ sont définis comme précédemment, que l'on cyclise par action d'une base, pour obtenir le composé de formule : 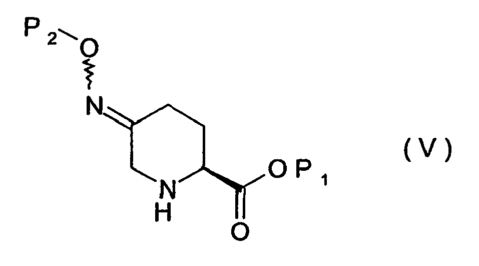 dans laquelle P₁ et P₂ sont définis comme précédemment, dont on réduit la fonction oxime par action d'un agent réducteur, pour obtenir le composé attendu de formule (I) que, si désiré, l'on salifie par action d'un acide.

Le composé bétacétosulfoxonium de formule (b) peut être obtenu au départ de l'acide (S) pyrroglutamique protégé de formule (a): 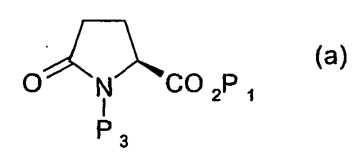 dans laquelle P₁ et P₃ sont définis comme précédemment, dont on ouvre le cycle par action d'iodure de triméthylsulfoxonium en présence de l'hydrure de sodium dans le tétrahydrofuranne.

Le groupement protecteur de la fonction acide carboxylique P₁ est notamment un reste d'ester d'alkyle, d'allyle, de benzyle ou p-nitrobenzyle, des restes équivalents connus de l'homme du métier pourraient bien entendu convenir également.

P₁ est de préférence un groupe benzyle.

Le groupement protection de l'azote P₃ forme notamment un carbamate et est de préférence un tert-butoxycarbonyle ou benzyloxycarbonyle, il peut aussi être un groupe électroattracteur tels que ceux connus de l'homme du métier référencés dans le « Greene » (Protective Groups in Organic Synthesis, 3^{ème} édition).

P₃ est de préférence un groupe tert-butoxycarbonyle.

Le groupement protecteur de l'hydroxylamine P₂ est notamment un reste benzyle ou allyle.

P₂ est de préférence un groupe benzyle.

Les conditions générant HCl et permettant de préparer le composé de formule (II) consistent de préférence à mettre en oeuvre le chlorure de lithium en présence d'un acide fort. On peut aussi utiliser simplement l'acide chlorhydrique. L'acide fort est par exemple l'acide chlorhydrique, l'acide sulfurique, un acide sulfonique tel que l'acide méthane sulfonique ou l'acide éthanesulfonique.

Dans des conditions préférées de mise en oeuvre de l'invention, on utilise le chlorure de lithium en présence d'acide méthanesulfonique.

On opère par exemple au sein d'un éther tel que le tétrahydrofuranne ou le dioxane, au sein du diméthylsulfoxyde ou au sein d'un ester tel que l'acétate d'éthyle.

La protection de la fonction cétone du composé de formule (II) est réalisée, sans isoler l'intermédiaire, en fonction du groupe protecteur choisi, dans des conditions connues de l'homme du métier.

La déprotection de la fonction amine est réalisée par action d'un acide, par exemple l'acide chlorhydrique, l'acide sulfonique, l'acide trifluoroacétique ou un acide alcanesulfonique. Selon la nature du groupe protecteur, ces conditions sont connues de l'homme du métier. Des conditions avantageuses consistent à utiliser un groupe protecteur tert-butoxycarbonyle et à le cliver par action d'acide méthane sulfonique. On peut opérer par exemple dans l'acétate d'éthyle.

L'α-chlorooxime protégée de formule (III) est de préférence mise en oeuvre sans être isolé, c'est-à-dire en solution dans le solvant de réaction. Il en est de même pour l'α-chlorooxime protégé de formule (IV).

La base utilisée pour cycliser le composé de formule (IV) est par exemple un hydroxyde, un carbonate ou bicarbonate alcalin, de préférence le bicarbonate de sodium, ou une base de type amine, par exemple la triéthylamine.

L'agent réducteur utilisé pour réduire la fonction oxime est par exemple un réactif de type borohydrure alcalin, diborane ou borane-pyridine en présence d'un acide, par exemple l'acide chlorhydrique. On peut opérer au sein d'un alcool tel que le méthanol ou l'éthanol, ou au sein d'un autre solvant organique tel que le dichlorométhane.

La salification du composé de formule (I) est le cas échéant réalisée par addition d'un acide en phase soluble au composé.

Parmi les sels d'acides des produits de formule (I), on peut citer entre autres, ceux formés avec les acides minéraux, tels que les acides chlorhydrique, bromhydrique, iodhydrique, sulfurique ou phosphorique ou avec les acides organiques comme l'acide formique, acétique, trifluoroacétique, propionique, benzoïque, maléique, fumarique, succinique, tartrique, citrique, oxalique, glyoxylique, aspartique, alcanesulfoniques, tels que les acides méthane et éthane sulfoniques, arylosulfoniques tels que les acides benzène et paratoluènesulfoniques.

Les sels sont de préférence ceux permettant une cristallisation dans des conditions aisées. Le sel d'acide oxalique est particulièrement préféré.

Des composés de type (a) et (b) ainsi que (II) dont les formules sont indiquées plus haut sont connus et on peut citer les références J.Chem. Soc. Chem. Comm. 1993, p.1434-1435 ou Tet.letters Vol. 29. N.18, p.2231-4 (1988).

Des essais de cyclisation des composés (b) et (II) ont été réalisés.

La cyclisation du composé (b) est possible mais implique l'utilisation d'un réactif à base de rhodium. La mise en oeuvre de ce type de réactif au niveau industriel n'est guère aisée et est très coûteuse. De plus, les rendements obtenus ne sont pas satisfaisants. Des alternatives au rhodium ont été recherchées sans succès.

La cyclisation du composé (II) quant à elle n'a pu être réalisée, la réactivité du groupe carbonyle étant probablement la cause de l'échec.

La présente invention fournit un procédé de préparation de l'intermédiaire de formule (I) dans des conditions particulièrement attractives, avec un rendement global de l'ordre de 70% et qui permet donc de surmonter l'échec rencontré précédemment.

Les composés de formule (III), (IV) et (V) obtenus lors de la mise en oeuvre du procédé sont nouveaux et constituent également l'un des objets de l'invention, à titre de composés industriels nouveaux et notamment d'intermédiaires nécessaires dans la préparation des composés de formule (I).

L'exemple suivant illustre l'invention.

### Exemple : (2S)-5-benzyloxyamino-pipéridine-2-carboxylique acide, benzylester et son oxalate

### Préparation : (5S)-5-tert-butyloxycarbonylamino-6-benzyloxy-2,6-dioxohexylide-diméthylsulfoxonium

L'hydrure de sodium (60 % dans l'huile, 15 g, 0,375 mole) est ajouté à une solution d'iodure de triméthylsulfoxonium dans le THF (0,4 1) agitée à température ambiante. Le mélange réactionnel est dilué avec le DMSO (0,5 1) puis est refroidi à -10 °C. Une solution de L-benzyl-N-Boc-Glutamate (100 g, 0,313 mole) dans le THF (0,351) est ajoutée. Le mélange réactionnel est réchauffé à 0 °C, agité pendant 45 minutes puis additionné à un mélange de chlorure d'ammonium (450 g), d'eau (1,51) et d'acétate d'éthyle (0,5 1) à 20 °C. La phase organique est isolée et lavée avec une solution de chlorure d'ammonium (180 g) dans l'eau (0,6 1) puis avec une solution de chlorure de sodium (200 g) dans l'eau (0,61). Les phases aqueuses sont extraites avec de l'acétate d'éthyle. Les phases organiques réunies sont séchées puis le produit est précipité en concentrant la solution sous pression réduite à 20 °C jusqu'à un volume de 0,41, puis en ajoutant du méthyl-tert-butyl éther (0,25 1). La suspension est refroidie à -10 °C, agitée pendant 2 h, filtrée puis lavée avec un mélange acétate d'éthyle-méthyl-tert-butyl éther (7 :3, 2x50 ml). Les cristaux sont séchés à 40 °C sous pression réduite pour donner le β-céto-sulfoxonium attendu (114,7 g, 279 moles, rendement 89 %).
RMN δ (400 MHz, MeOD) 1.49 (s, 9H, C₄H₉), 1.92 (m, 1H), 2.13 (m, 1H), 2.26 (m, 2H), 3.50 (s, 6H, S(CH₃)₂), 4.20 (m, 1H), 5.25 (m, 2H), 7.45 (m, 5H, C₆H₅).

### Stade A : (S)-5-(benzyloxyimino)-2-tert-butyloxycarbonylamino-6-chloro-hexa-noïque acide, benzyl ester (E+Z)

L'acide méthanesulfonique (29,3 g, 0,305 mole) est ajouté lentement à un mélange de β -cétosulfoxonium (114 g, 0,277 mol) et de chlorure de lithium (13,3 g, 0,314 mole) dans le THF (1,71 ml) à température ambiante. Le mélange réactionnel est chauffé à 50 °C pendant 5 heures puis refroidi à température ambiante. L'hydrochlorure de benzylhydroxylamine (46,4 g, 0,291 mole) et l'acétate de sodium (29,6 g, 0,361 mole) sont ajoutés. Le milieu réactionnel est dilué avec de l'acétate d'éthyle (0,51) et de l'eau (0,5 1) puis agité à température ambiante pendant 18 heures. La phase organique est isolée, concentrée jusqu'à un volume de 0,4 1 puis lavée avec une solution de chlorure de sodium (25 g) dans l'eau (0,25 1). La phase aqueuse est séparée puis extraite avec l'acétate d'éthyle (0,2 1). Les phases organiques sont réunies et agitées 1 heure avec du sulfate de sodium (100 g). Le mélange est filtré et rincé avec de l'acétate d'éthyle (2 x 0,1 1). La solution d'α-chlorooxime est conservée au réfrigérateur pour l'étape suivante où elle est utilisée telle quelle. (130,8 g, 0,275 mole, rendement 99,3 %).
RMN δ (400 MHz, CDCl₃) 1.45 (s, 9H, C₄H₉), 1.96 (m, 1H, CH_{A}H_{B}), 2.16 (m, 1H, CH_{A}H_{B}), 2.47 (m, 2H, CH₂), 4.06 et 4.20 (2s, 2H, CH₂Ph), 4.38 (m, 1H), 5-5.4 (m, 4H), 7.35 (m, 10H, 2×C₆H₅); m/z (+ESI, LCMS) délecté 475.0 [MH⁺].

### Stade B : (S)-5-(benzyloxyimino)-pipéridine-2-carboxylique acide, benzyl ester (E+Z)

La solution d'α-chlorooxime (0,275 mol) est séchée par distillation azéotropique jusqu'à un volume de 0,5 1 puis diluée avec l'acétate d'éthyle (0,5 1). La solution est refroidie à 0 °C. L'acide méthanesulfonique (136 g, 1,42 mole) est ajouté en 15 minutes. Le mélange réactionnel est chauffé à 40 °C pendant 1 heure puis refroidi à température ambiante avant d'être ajouté à une solution de bicarbonate de sodium (279 g, 3,40 moles) dans l'eau (11). Le mélange réactionnel est chauffé à 50 °C pendant 3 heures puis refroidi à température ambiante. La phase organique est isolée puis lavée avec une solution de chlorure de sodium (50 g) dans l'eau (0,5 1). Les phases aqueuses sont extraites avec de l'acétate d'éthyle (0,51). Les phases organiques sont réunies. La solution est mélangée avec de la silice (100 g) puis agitée pendant 20 minutes. La solution est filtrée puis lavée avec de l'acétate d'éthyle. La solution de pipéridinéoxime est concentrée jusqu'à un volume de 0,21 puis conservée au réfrigérateur pour l'étape suivante. (88,8 g, 0,262 mole, rendement 95,3 %).

Les isomères E et Z de l'oxime sont séparés par chromatographie puis analysés par RMN ;
RMN Isomère présumé E δ (400 MHz, CDCl₃) 1.8 (m, 1H), 2.25 (m, 3H), 3.15 (m, 1H), 3.35 (d, 1H), 3.62 (d, 1H), 3.64 (d, 1H), 5.1 (s, 2H, CH₂Ph), 5.2 (s, 2H, CH₂Ph), 7.37 (m, 10H, 2×C₆H₅);
RMN Isomère présumé Z δ (400 MHz, CDCl₃) 1.9 (m, 1H), 2.20-2.60 (m, 4H), 3.35 (d, 1H), 3.64 (d, 1H), 4.3 (d, 1H), 5.1 (s, 2H, CH₂Ph), 5.2 (s, 2H, CH₂Ph), 7.37 (m, 10H, 2×C₆H₅).

### Stade C : (2S)-5-benzyloxyamino-pipéridine-2-carboxylique acide, benzyl ester et son oxalate (mélange (2S,5R) et (2S,5S) ∼ 50/50).

L'acétate d'éthyle, dans lequel la pipéridinoxime obtenue au stade B est en solution est remplacé par du méthanol par distillation puis dilution jusqu'à un volume de 0,21. La solution de pipéridinoxime (0,261 mole) est ajoutée en 30 minutes à une solution de HCl (1,32 mole) dans le méthanol (0,311) à 0 °C. le borane pyridine (45,4 g, 0,49 mole) est ajouté en 4 heures au milieu réactionnel à 0 °C. Le milieu est réchauffé à température ambiante puis agité pendant une nuit. La solution est concentrée jusqu'à un volume de 0,18 1 puis diluée avec du dichlorométhane (0,36 1) et de l'eau (0,36 1). Une solution aqueuse de soude à 50 % est ajoutée jusqu'à pH 7. La phase aqueuse est séparée puis extraite avec du dichlorométhane (0,27 1). La phase organique est lavée à l'eau. La solution de (2S)-5-benzyloxyamino-pipéridine-2-carboxylique acide, benzyl ester est reprise et le dichlorométhane est remplacé par l'acétate d'éthyle par distillation puis dilution jusqu'à un volume de 0,72 1. Une solution d'acide oxalique (23,78 g) dans l'acétate d'éthyle (0,27 1) est ajoutée en 40 minutes. La suspension est agitée pendant 2 heures à température ambiante, filtrée, lavée à l'acétate d'éthyle (3 x 90 ml) et séchée à 30 °C pour donner l'oxalate d'oxyamine sous forme de poudre (95,32 g, 0,221 mole, rendement 85 %).

## Revendications

1. Procédé de préparation du composé de formule (I) 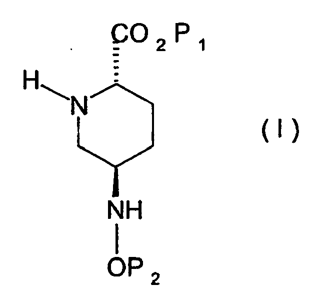 dans laquelle P₁ et P₂ représentent des groupements protecteurs des fonctions acide carboxylique et oxyamine, **caractérisé en ce que** l'on traite le composé bétacétosulfoxonium de formule : 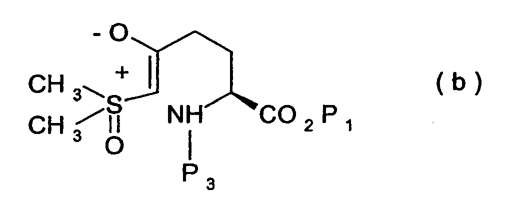 dans laquelle P₁ est tel que défini précédemment et P₃ représente un groupement protecteur d'amine, avec un réactif générant HCl, pour obtenir le composé de formule : 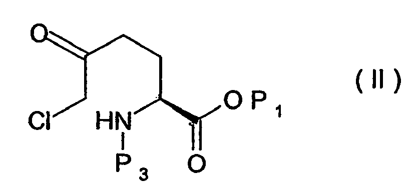 dans laquelle P₁ et P₂ sont définis comme précédemment, que l'on traite sans l'isoler par un dérivé d'hydroxylamine, pour obtenir le composé de formule : 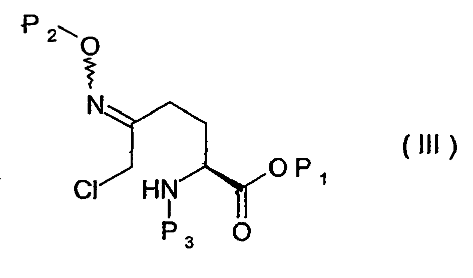 dans laquelle P₁ et P₃ sont définis comme précédemment et P₂ représente un groupement protecteur de l'oxime, dont on déprotège l'amine par action d'un acide, pour obtenir le composé de formule : 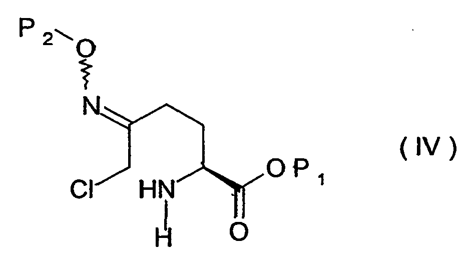 dans laquelle P₁ et P₂ sont définis comme précédemment, que l'on cyclise par action d'une base, pour obtenir le composé de formule : 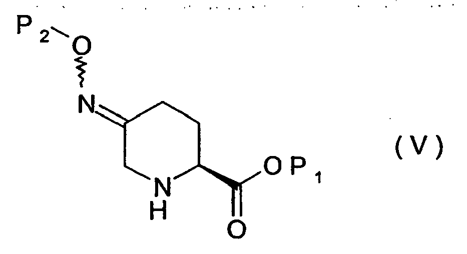 dans laquelle P₁ et P₂ sont définis comme précédemment, dont on réduit la fonction oxime par action d'un agent réducteur, pour obtenir le composé attendu de formule (I) que, si désiré, l'on salifie par action d'un acide.

2. Procédé selon la revendication 1, **caractérisé en ce que** P₁ représente un groupe benzyle.

3. Procédé selon la revendication 1 ou 2, **caractérisé en ce que** P₃ représente un groupe tert-butoxycarbonyle.

4. Procédé selon l'une quelconque des revendications 1 à 3, **caractérisé en ce que** P₂ représente un groupe benzyle.

5. Procédé selon l'une quelconque des revendications 1 à 4, **caractérisé en ce que** l'on traite le composé de formule (b) par le chlorure de lithium en présence d'acide méthane sulfonique.

6. Procédé selon l'une quelconque des revendications 1 à 5, **caractérisé en ce que** l'on déprotège l'amine du composé de formule (III) par action de l'acide méthane sulfonique, sans isolement préalable de ce composé.

7. Procédé selon l'une quelconque des revendications 1 à 6, **caractérisé en ce que** l'on effectue la cyclisation du composé de formule (IV) par action du bicarbonate de sodium.

8. Un composé de formule (III) : 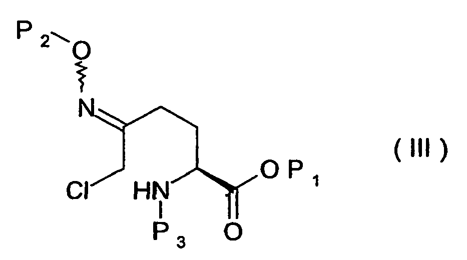 dans laquelle P₁, P₂ et P₃ sont définis comme à la revendication 1.

9. Un composé de formule (IV) : 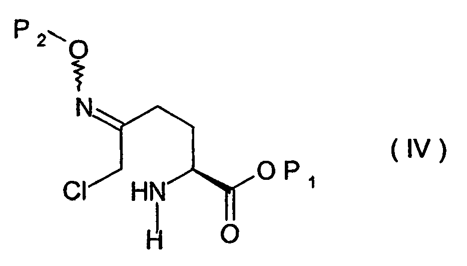 dans laquelle P₁ et P₂ sont définis comme à la revendication 1.

10. Un composé de formule (V) : 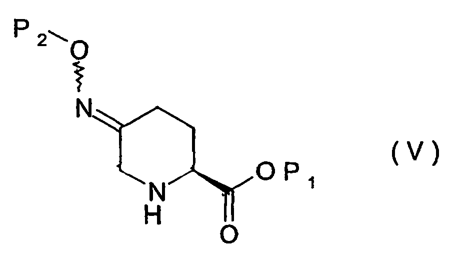 dans laquelle P₁ et P₂ sont définis comme à la revendication 1.

## Claims

1. A method for preparing the compound of formula (I) 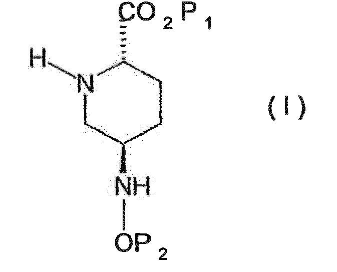 wherein P₁ and P₂ represent groups protecting the carboxylic acid and oxyamine functions, **characterized in that** the beta-ketosulfoxonium compound of formula: 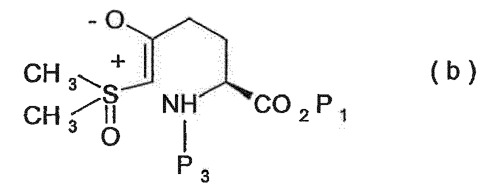 wherein P₁ is defined here above and P₃ represents an amine protecting group, is treated with a reagent generating HCl, in order to obtain the compound of formula: 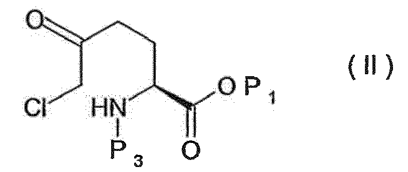 wherein P₁ and P₂ are as defined here above, which is treated without isolating it, with a hydroxylamine derivative, in order to obtain the compound of formula: 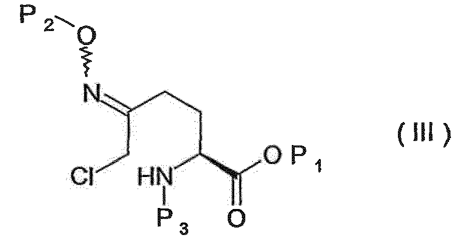 wherein P₁ and P₃ are as defined here above and P₂ represents a protecting group of the oxime, the amine of which is de-protected by the action of an acid, in order to obtain the compound of formula: 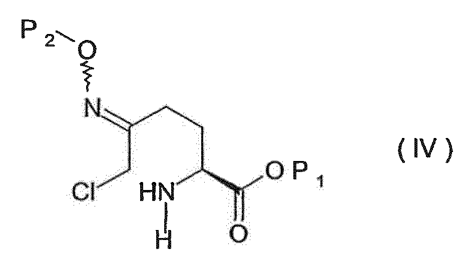 wherein P₁ and P₂ are defined as here above, which is cyclized by the action of a base, in order to obtain the compound of formula: 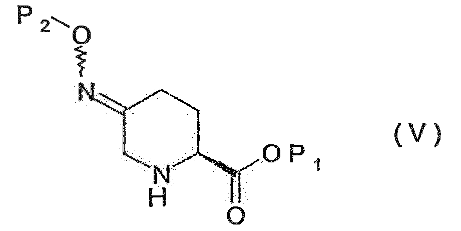 wherein P₁ and P₂ are defined as here above, the oxime function of which is reduced by the action of a reducing agent, in order to obtain the expected compound of formula (I) which, if desired, is salified by the action of an acid.

2. The method according to claim 1, **characterized in that** P₁ represents a benzyl group.

3. The method according to claim 1 or 2, **characterized in that** P₃ represents a tert-butoxy-carbonyl group.

4. The method according to any of claims 1 to 3, **characterized in that** P₂ represents a be group.

5. The method according to any of claims 1 to 4, **characterized in that** the compound of formula (b) is treated with lithium chloride in the presence of methane sulfonic acid.

6. The method according to any of claims 1 to 5, **characterized in that** the amine of the compound of formula (III) is de-protected by the action of methane sulfonic acid, without prior isolation of this compound.

7. The method according to any of claims 1 to 6, **characterized in that** the cyclization of the compound of formula (IV) is carried out by the action of sodium bicarbonate.

8. A compound of formula (III): 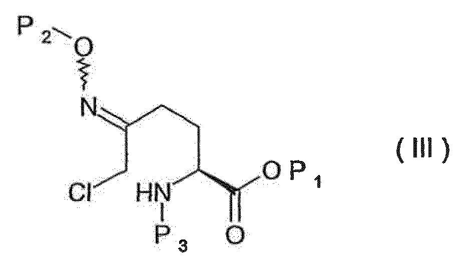 wherein P₁, P₂ and P₃ are as defined in claim 1.

9. A compound of formula (IV): 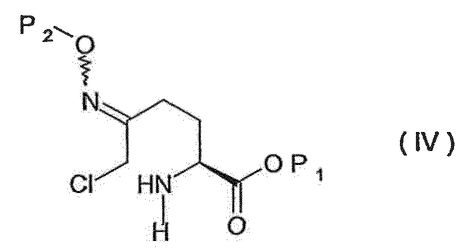 wherein P₁ and P₂ are as defmed in claim 1.

10. A compound of formula (V): 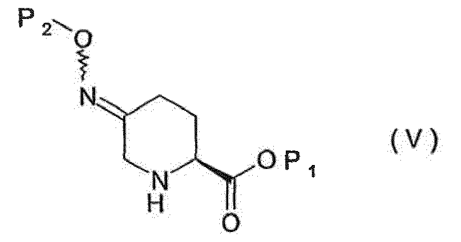 wherein P₁ and P₂ are as defined in claim 1.

## Patentansprüche

1. Verfahren zur Herstellung der Verbindung nach Formel (I) 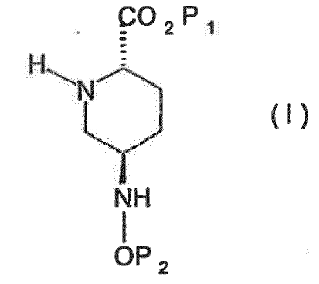 in welcher P₁ und P₂ Schutzgruppen für die Carbonsäure- und Oxyaminfunktionen darstellen, **dadurch gekennzeichnet, dass** die beta-Ketosulfoxoniumverbindung nach Formel: 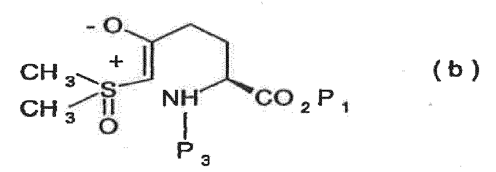 in welcher P₁ der obigen Begriffsbestimmung entspricht und P₃ eine Schutzgruppe für ein Amin darstellt, mit einem HCl-erzeugenden Reagenz behandelt wird, um die Verbindung nach Formel: 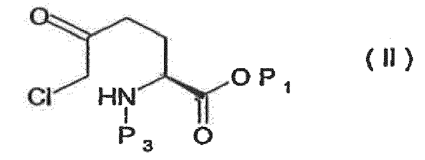 in welcher P₁ und P₂ der obigen Begriffsbestimmung entsprechen, zu erhalten, die ohne Isolierung mit einem Hydroxylaminderivat behandelt wird, um die Verbindung nach Formel 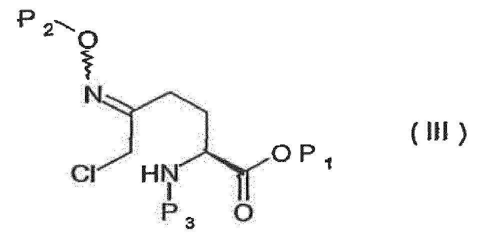 in welcher P₁ und P₃ der obigen Begriffsbestimmung entsprechen und P₂ eine Schutzgruppe für das Oxim darstellt, zu erhalten, woraufhin die Amin-Schutzgruppe derselben durch Einwirkung einer Säure entfernt wird, um die Verbindung nach Formel: 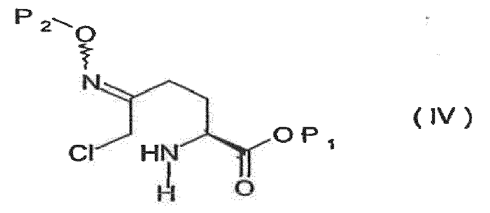 in welcher P₁ und P₂ der obigen Begriffsbestimmung entsprechen, zu erhalten, woraufhin diese durch Einwirkung einer Base einen Ringschluss erfährt, um die Verbindung nach Formel: 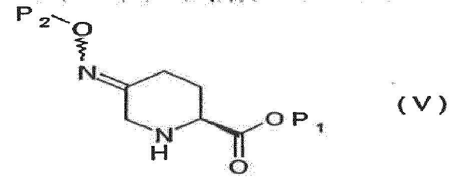 in welcher P₁ und P₂ der obigen Begriffsbestimmung entsprechen, zu erhalten, woraufhin deren Oxim-Funktion durch Einwirkung eines Reduktionsmittels reduziert wird, um die Zielverbindung nach Formel (I) zu erhalten, welche gegebenenfalls durch die Einwirkung einer Säure in die Salzform überführt werden kann.

2. Verbindung nach Anspruch 1, **dadurch gekennzeichnet, dass** P₁ eine Benzylgruppe darstellt.

3. Verbindung nach Anspruch 1 oder 2, **dadurch gekennzeichnet, dass** P₃ eine tert.-Butoxycarbonylgruppe darstellt.

4. Verfahren nach irgendeinem der Ansprüche 1 bis 3, **dadurch gekennzeichnet, dass** P₂ eine Benzylgruppe darstellt.

5. Verfahren nach irgendeinem der Ansprüche 1 bis 4, **dadurch gekennzeichnet, dass** die Verbindung nach Formel (b) in Gegenwart von Methansulfonsäure mit Lithiumchlorid behandelt wird.

6. Verfahren nach irgendeinem der Ansprüche 1 bis 5, **dadurch gekennzeichnet, dass** die Amin-Schutzgruppe der Verbindung nach Formel (III) durch Einwirkung von Methansulfonsäure entfernt wird, ohne vorherige Isolierung dieser Verbindung.

7. Verfahren nach irgendeinem der Ansprüche 1 bis 6, **dadurch gekennzeichnet, dass** bei der Verbindung nach Formel (IV) der Ringschluss durch Einwirkung von Natriumhydrogencarbonat hergestellt wird.

8. Verbindung nach Formel (III): 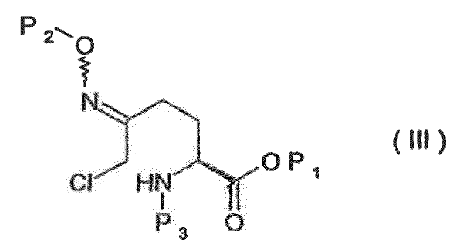 in welcher P₁, P₂ und P₃ den Begriffsbestimmungen in Anspruch 1 entsprechen.

9. Verbindung nach Formel (IV) 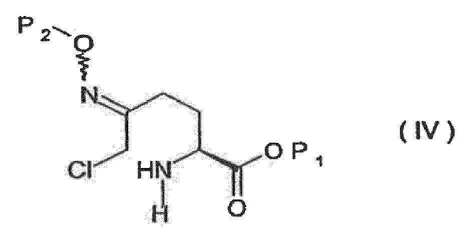 in welcher P₁ und P₂ den Begriffsbestimmungen in Anspruch 1 entsprechen.

10. Verbindung nach Formel (V): 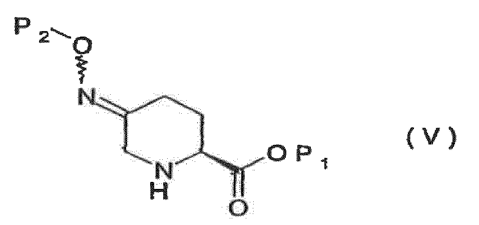 in welcher P₁ und P₂ den Begriffsbestimmungen in Anspruch 1 entsprechen.
